# EUROPEAN PATENT SPECIFICATION

(11) **EP 1 542 788 B1**
(45) Date of publication and mention of the grant of the patent: **20.09.2006**
(21) Application number: 03798180.0
(22) Date of filing: 24.09.2003
(51) Int. Cl.: B01D 69/02, B01D 67/00, A61K 9/00, B01D 61/42, B01D 71/02

(54) **METHOD FOR PRODUCING A NANODEVICE FOR CONTROLLED CHARGED PARTICLE FLOW**
VERFAHREN ZUR HERSTELLUNG EINER NANOVORRICHTUNG FÜR EINE KONTROLLIERTE STRÖMUNG GELADENER TEILCHEN
PROCEDE DE PRODUCTION D'UN NANO-DISPOSITIF POUR L'ECOULEMENT CONTROLE DE PARTICULES CHARGEES

(30) Priority: 25.09.2002 DE 10244914; 25.09.2002 US 254947
(43) Date of publication of application: 22.06.2005
(73) Proprietor: Gesellschaft für Schwerionenforschung mbH, 64291 Darmstadt (DE)
(72) Inventor: SIWY, Zuzanna, 64291 Darmstadt (DE); BEHRENDS, Jan, 80799 München (DE); FERTIG, Niels, 80335 München (DE); FULINSKI, Andrzej, PL- Krakau (PL); MARTIN, Charles, R., Gainesville, FL (US); NEUMANN, Reinhard, 69221 Dossenheim (DE); TRAUTMANN, Christina, 64291 Darmstadt (DE); TOIMIL MOLARES, Eugenia, 64291 Darmstadt (DE)
(74) Representative: Boeters, Hans Dietrich
(86) International application number: PCT/EP2003/010631
(87) International publication number: WO 2004/028673

(56) References cited:
- EP-A- 0 252 545
- WO-A-02/20877
- WO-A-02/36230
- DE-A- 19 853 286
- US-A- 5 593 560
- US-A- 5 736 050
- PATENT ABSTRACTS OF JAPAN vol. 012, no. 037 (C-473), 4 February 1988 (1988-02-04) -& JP 62 186904 A (KIYOSHI SUGAI), 15 August 1987 (1987-08-15) -& DATABASE WPI Section Ch, Week 198738 Derwent Publications Ltd., London, GB; Class J01, AN 1987-268003 XP002226679 & JP 62 186904 A (SUKAI K), 4 February 1988 (1988-02-04)
- PATENT ABSTRACTS OF JAPAN vol. 002, no. 126 (C-025), 21 October 1978 (1978-10-21) & JP 53 091079 A (TOKUYAMA SODA CO LTD), 10 August 1978 (1978-08-10) -& DATABASE WPI Section Ch, Week 197837 Derwent Publications Ltd., London, GB; Class J01, AN 1978-66151A XP002269273 & JP 53 091079 A (TOKUYAMA SODA KK)

## Description

The present invention relates to a method for producing a nano-device for controlling the flow of charged particles in electrolytes.

In many electrolytic systems the problem of controlling ion flow, rather than flow of electrons, is crucial. For the flow of electrons diodes and transistors are the basic elements controlling, switching on and off and amplifying the signal. For the ionic current there exist very limited possibilities to tune the ion flow. From German patent application 100 44 565.9 an electrochemical rectifier is known, based on preparation of asymmetric pores in a polymeric foil. One mode of operation entails applying a constant voltage across a membrane with asymmetric pores, however changing the current would require changing the concentration and/or the pH-value of the electrolyte. Since this changing of concentration and/or pH-value is time consuming and disturbs the condition of operation, this option may not be applicable for a given system.

Document WO 02/20877 A1 describes a method for etching at least one pore along an ion track in a membrane.

The abstract of JP 62186904 describes a membrane with fine pores and an electrically conductive layer on one side of the membrane.

Therefore, it is the object of the present invention to provide an improved method for producing a nanodevice.

An apparatus having a nanodevice for controlling the flow of charged particles in electrolytes is provided, comprising an electrolytic bath container, divided by a polymeric foil into a first and a second compartment. Each compartment comprises an electrode connected to a voltage supply. Further, the nanodevice comprises at least one preferentially asymmetric pore forming a via hole through said foil, wherein said pore provides a narrow opening of a diameter in the range of several nanometers down to about one nanometer on a front side of said foil and a wide opening in the range of several ten nanometers up to several hundred nanometers on a back side of said foil.

The polymeric foil is covered on its front side by an electrically conductive layer surrounding said narrow opening. A gate voltage supply is connected to said electrically conductive layer on said front side of said foil controlling the flow of charged particle within said nanodevice from said first compartment to said second compartment and vice versa.

This nanodevice has the advantage to control or to switch on and off a charged particle flow of heavy ions, ions of macromolecules, ions of bio-molecules, ionized dimeric, ionized oligomeric or ionized polymeric DNA or ionized insulin. In such a nanodevice with such a pore the spatial distribution of electric potential inside the pore is changed by the gate voltage on the electrically conductive layer of the polymeric foil in order to advantageously tune the flow of ion through the pore. The electrically conductive layer forms a gate close to the narrow opening of the conical, funnel-like, or trumpet-like pore or at the entrance of a cylindrical pore, where the pore has its highest resistance. Such a gating ion flow would allow to control ionic current through the asymmetric pore.

The polymeric foil comprises polyethylene terephthalate, polyimide or polycarbonate. These materials have the advantage, that an ion trace can be performed through said foil by a high accelerated ion like bismuth. Such a trace across a foil material can be etched in an electrolytic cell consisting of two cell halves filled with an electrolytic solution and being divided by said foil comprising said ion trace. These materials have further the advantage that a nanodevice made of same is cation selective.

According to another example, a gold layer as electrically conductive layer surrounding said narrow opening of said front side. Such a gold layer as a gate electrode has the advantage, that it is resistive against corrosion and oxidation. Therefore, such gold layer can be used in different electrolytic bathes to control and/or switch on an off a flow of charged particles.

Another preferred material for the gate electrode is a semiconductor like indium oxide or ITO. Indium oxide has the advantage, that it is erosion and oxidation resistant in almost any electrolytic bath.

The back side of said foil may be covered by an electrically conductive layer surrounding said wide opening. Such a second metal layer on the back side of the polymeric foil enables the nanodevice to make the ion current changes finer and better controlled.

The nanodevice is not limited either to polymer films or to a gold layer, since any asymmetric and charged nanopores together with a conductive layer, which can be charged by a voltage, can be potentially used to accomplish the present invention.

A membrane with asymmetric pores enables an optimal control of the particle flow, offering at the same time higher mass flow than a corresponding cylindrical pore of the same effective diameter. The principles of functioning of the nanodevice are valid however also for symmetric, for example, cylindrical pores with a metal (or semiconductor) layer sputtered on one side of the membrane. This broadens immensely the possibilities of manufacturing a device of this kind, because also commercially available membranes with cylindrical pores of any material (e.g. polymer, anodic alumina) can be used. By applying voltage to the layer, it makes the system asymmetric as far as distribution of electric potential inside the pore is concerned.

Covering the conductive layer by a thin insulating layer that is chemically inactive in an electrolyte solution limits the influence of the "gate" potential to the entrance of the pore with said layer. This is expected to improve the control over the charged particle flow.

Ion flow control is possible also with pores of diameter up to hundreds of nm. In said large-pore set-up the layer representing the third electrode is preferentially made of a non-insulating i.e. electrically conductive material. The pores are not selective with respect to different ion species, and the mechanism of ion flow control differs from that in very narrow pores. The potential on the conductive layer (U₂) superimposes the potential difference applied across the membrane (U₁), which for given voltage configurations provides the possibility of enhancing or stopping anions (cations). This was demonstrated with a membrane containing 10⁷ pores/cm² and methylene blue dye.

One preferred mode of operation of the nanodevice is to provide a direct current voltage supply for the electrolytic bath and a direct current voltage supply for the gate voltage. Another preferred mode of operation is to apply an alternating voltage to the gate, which enables to achieve a pulse-like flow of charged molecules through said nanodivice with at least one asymmetric pore.

Preferred applications of the apparatus having a nanodevice are
1. Separation processes for the pharmaceutical industry,
2. Controlled release of bio-molecules like insulin,
3. Voltage-controlled nanosystems,
4. Tuning of the ion current signal,
5. Gating of ionic bio-molecule in microfluicid lab-on-a-chip devices.

The invention is related to a method for producing a nanodevice. Such a method comprises the steps of:
- irradiating a membrane of a polymeric foil by at least one high accelerated ion to form an ion trace through said foil;
- etching said ion trace from a back side of said foil toward a front side of said foil to form a pore having a wide opening on said back side in the range several ten nanometers up to several hundred nanometers and a narrow opening on said front side in the range of several nanometers down to about one nanometer;
- drying said etched foil;
- depositing an electrically conductive layer on said front side by diminishing the narrow opening;
- reopen said narrow opening to a predetermined diameter by etching said conductive layer from its back side.

This method has the advantage that a conical, a funnel-like or a trumpet-like nanopore is performed along the ion trace through the polymeric foil dependent on the parameters of an electrolytic process in an electrolytic cell consisting of two cell halves filled with an electrolytic solution.

In a preferred method at least a single bismuth ion is accelerated to an energy in the range of 10 to 15 MeV and irradiated toward said polymeric foil to form said ion trace. This bismuth ion is particularly advantageous if applied to foils made of polyethylene terephthalate, polyimide and/or polycarbonate. This trace is preferably etched by a caustic solution, where such a solution can comprise 9m NaOH. This caustic solution has the advantage that the ion trace can be etched at room temperature. After etching along the ion trace an asymmetric pore, the front side of the foil is deposited with a electrically conductive layer like a gold or indium oxide layer by a sputter technique.

To increase the adhesiveness of an electrically conductive layer like a gold layer or a semiconductor layer on the polymer surface of the foil, it is an advantage to roughened the surface of the polymer foil before etching the irradiated film.

During said deposition of a metal or a semiconductor on said front side the narrow opening is diminished. To reopen said narrow opening a piece of a conductive tape is attached to cover the conductive layer. After that it is an advantage to reinsert said foil with its electrically conductive layer and said piece of a conductive tape in an electrolytic cell, wherein the two cell halves are filled with potassium fluoride, whilst a conductive tape stays attached to the conductive layer.

Further embodiments, features and advantages of the invention are now discussed with reference to the attached drawings.
- Fig. 1: shows a schematic drawing of an apparatus having a nanodevice for controlling the flow of charged particles in electrolytes.
- Fig. 2: shows principles of the functioning of the nanodevice for controlling the flow of charged particles in electrolytes.
- Fig. 3: shows a scheme of an experimental set-up for evaluating the performance of the nanodevice.
- Fig. 4: shows current-voltage characteristics of a single conical pore in a polyethylene terephthalate (PET) foil with a gold layer on the side of the small or narrow opening of a pore, applying 0.1m KF on both sides of the membrane foil.
- Fig. 5a,b: show examples of time series without and with applied "gate voltage" for two directions of the potassium ion flow.

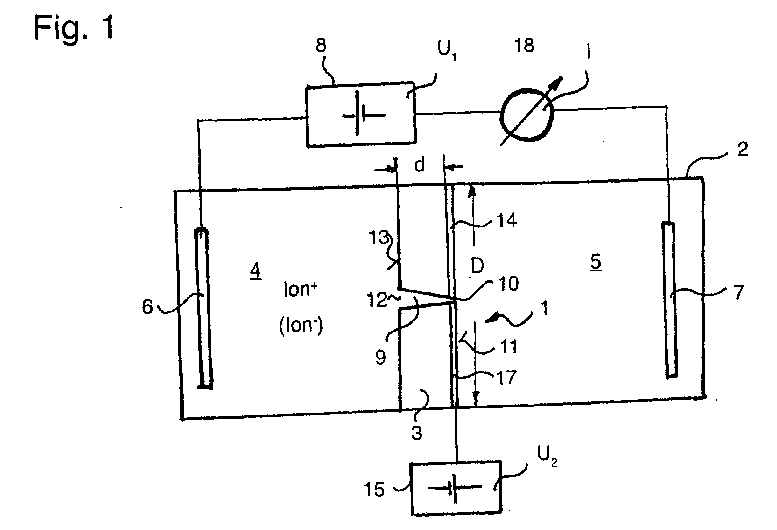

Figure 1 shows an embodiment of an apparatus having a nanodevice 1 for controlling the flow of charged particles (ion⁺ or ion⁻) in an electrolyte. This device comprises an electrolytic bath container 2 divided by a polymeric foil 3 into a first 4 and a second 5 compartment. Each compartment 4 and 5 comprises an electrode 6 and 7 connected to a voltage supply 8, which supplies in this embodiment a direct current voltage U₁. The electrolytic current I is measured by a current meter 18.

If the potential of electrode 6 is positive, positive charged particles like ion⁺ are forced through the asymmetric pore 9 of the foil 3 from a wide opening 12 on the back side 13 of the foil 3 to a narrow or small opening 10 on the front side of said foil 3. The foil itself is a circular disc having a diameter D of about 30 mm and a thickness d of 12 µm. The material of the foil in this embodiment is polyethylene terephthalate which was irradiated in its center with a single bismuth ion of 11.4 MeV specific energy and etched from one side in a 9m NaOH at room temperature to form said pore 9.

The polymeric foil 3 is covered on the front side 11 by a gold layer surrounding the narrow opening 10 of said pore 9. This gold layer functions as a gate electrode 17, which is supplied by a gate voltage U₂ supply 15. If this gate voltage U₂ is negative, the charged particles in the first compartment 4 like ion⁺ are accelerated, so that the flow through the nanodivice 1 is increased toward the second compartment 5. When Increasing U₂ toward a positive gate voltage the flow of positive charged particles (ion⁺) is decreased and can even be switched off.

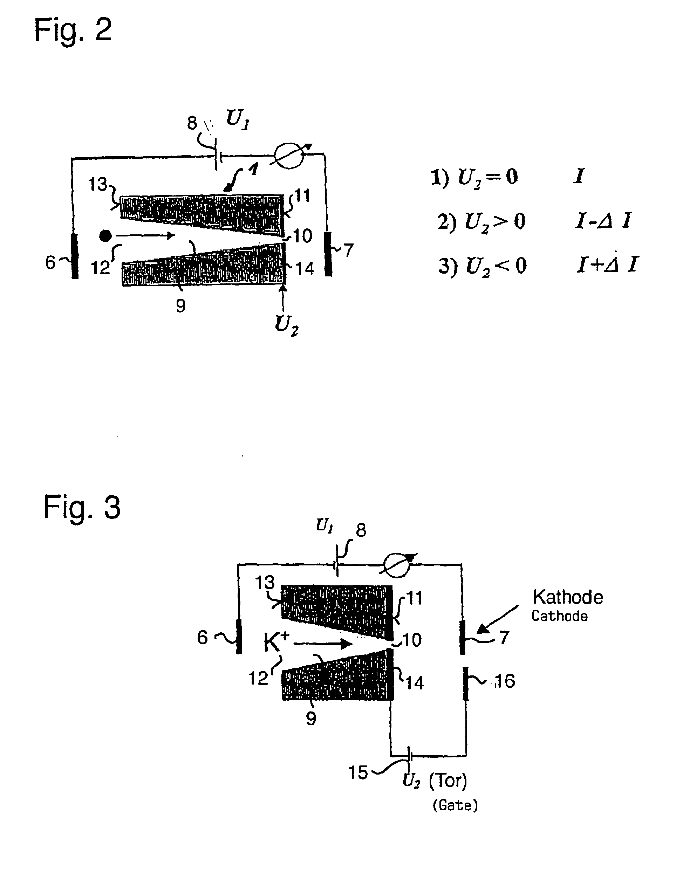

Figure 2 shows principles of the functioning of the nanodevice 1 for controlling the flow of charged particles (ion⁺, ion⁻) in electrolytes. Components with the same functions as in Figure 1 are characterized by the same reference signs and an explanation of same is omitted.

Figure 2 shows in detail the principles of operating a device shown in Figure 1. A thin layer 14 of metal or semiconductor is sputtered on the front side 11 of the foil 3 having such narrow openings 10. This layer 14 can be charged via an independent electric circuit U₂. If the pore is very narrow, the passage of the ions through the pore will be influenced by such a "gate". The pore produced by a track-etching technique in a foil 3 made of polyethylene terephthalate, polyimide or polycarbonate are negatively charged due to formation of carboxylate groups, therefore they are cation-selective. This means that cations are the main charge carriers.

Applying a positive voltage U₂ slows down the flow of cations observed as lower current. Applying a negative voltage U₂ has an opposite effect, the current will be larger. Accordingly, this device will be the first device, which can control the ion flow, based on asymmetry of electric potential introduced by the conical, funnel-like or trumpet-like shape of a charged nanopore in combination with applying locally electric fields. Since this layer can be charged positively or negatively by means of the voltage applied via an independent circuit, this results in changes of the profile of the electric potential at the pore constriction, which influences the ion current flow.

Figure 3 shows a scheme of an experimental set-up of evaluating the performance of the nanodevice 1. Components having the same functions as in Figure 1 or in Figure 2 are characterized by the same reference signs and an explanation of same is omitted.

The evaluation of the performance of the nanodevice 1 is made for a single pore within a 12 µm thick circular disc of 30 mm diameter. For the purpose of etching such a pore in a 9m NaOH this disc or membrane is inserted between two halves of an electrolytic cell and sealed hermetically by applying pressure onto the two cell halves. When the etching process is completed, the polymer foil 3 is removed from the cell and dried. In a next step, a gold layer 14 is sputtered on the front side 11 with its narrow opening 10. Then, a piece of a conductive tape is attached to the front side onto the gold layer. Now, the foil is inserted back into the electrolytic cell, which chambers are now filled with potassium fluoride.

The current through the pore 9 is measured with electrodes of Ag/AgCl. An independent circuit is built, which applies a voltage to the gold layer via the conductive tape. The scheme of the experimental set-up is shown in Figure 3. The use of fluoride ions in a KF solution increases the effect of the applied voltage during the electrolytic procedure. F⁻ ions do not adsorb to the gold layer.

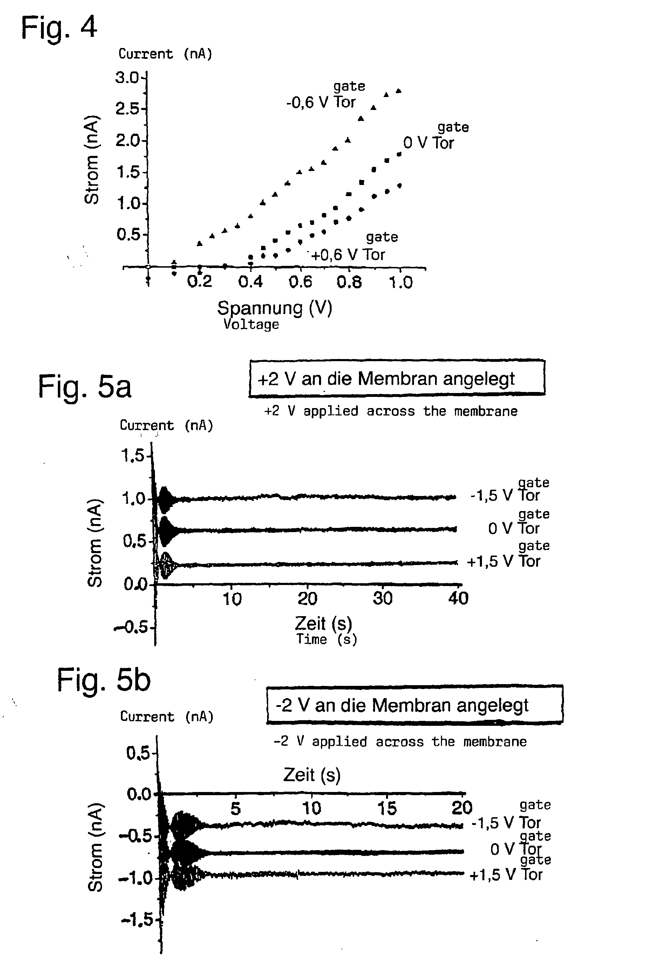

Figure 4 shows a current-voltage characteristic of a single conical pore 9 in a PET-foil. The abscissa of said diagram shows the voltage U₁ in V and the ordinate shows the current in nA. The dotted curves shows the effect of the parameters: +0.6 V at the metal gate electrode, 0V at the metal gate and -0.6 V at the metal gate. As one can see from this evaluation it is possible to enhance the current through the asymmetric pore at a voltage U₁ of 0.4 V by a gate voltage U₂ of -0.6 V up to around 1 nA, whilst with a positive gate voltage of 0.6 V the ionic current is decreased to 0 or shut off.

Figures 5a and 5b show examples of time series without and with applying a gate voltage for two directions of the potassium ion flow. If a voltage of +2V is applied across the membrane a voltage of +1.5V at the gate will decrease the current drastically, whilst a gate voltage of -1.5 will increase the current up to 1nA. In these diagrams of Figures 5a and 5b the abscissa shows the time in seconds and one can see that after three seconds the current is relatively constant.

In Figure 5b the voltage across the membrane or across the pore is changed to -2V, so that the current is also negative. By applying negative gate voltage of about -1.5V the current is decreased, whilst applying a positive gate voltage of 1.5V the current is increased to -1nA. This diagrams show that this nanodevice is quite sensitive and works like a triode for ions in an electrolytic bath.

### List of reference signs

- 1: nanodevice
- 2: electrolytic bath container
- 3: polymeric foil
- 4: first compartment
- 5: second compartment
- 6: electrode
- 7: electrode
- 8: voltage (U₁) supply
- 9: asymmetric pore
- 10: narrow opening
- 11: front side
- 12: wide opening
- 13: back side
- 14: electrically conductive layer
- 15: gate voltage (U₂) supply
- 16: electrode
- 17: gate electrode
- 18: current meter

## Claims

1. A method for producing a nanodevice (1) comprising the steps of:
- irradiating a polymeric foil (3) by at least one highly accelerated ion to form an ion trace through said foil;
- etching said ion trace from a first side (13) of the polymeric foil (3);
- drying said etched foil (3);
- depositing an electrically conductive layer (14) on a second side (11) of the polymeric foil thereby diminishing an opening (10) at the second side ot the polymeric foil;
- reopen said opening (10) to a predetermined diameter by etching said conductive layer (14) from the first side (13).

2. The method according to claim 1, wherein a single bismuth ion is accelerated to an energy in the range of 10 to 15 MeV and irradiated toward said polymeric foil (3) to form said ion trace.

3. The method according to claim 1 or claim 2, wherein said ion trace is etched by a caustic solution.

4. The method according to claim 3, wherein said caustic solution comprises 9m NaOH.

5. The method according to one of the claims 1 to 4, wherein said ion trace is etched at room temperature.

6. The method according to one of the claims 1 to 5, wherein said deposition is carried out by sputtering a metal or a semiconductor on to said second side (11).

7. The method according to one of the claims 1 to 6, wherein said second side (11) of said foil (3) is roughened before etching said ion trace.

8. The method according to one of the claims 1 to 7, wherein said foil is inserted in an electrolytic cell consisting of two cell halves filled with a KF solution and being divided by said foil to etch said ion trace, wherein said foil is sealed hermetically to the two cell halves.

9. The method according to one of the claims 1 to 8, wherein a conductive tape is attached to the conductive layer (14) before said reopening of said opening (10) is performed.

10. The method according to claim 9, wherein said foil covered on its second side (11) by a conductive tape is reentered to said electrolytic cell, which cell halves are now filled with NaF.

## Patentansprüche

1. Verfahren zur Herstellung einer Nanovorrichtung (1) umfassend die Schritte:
- Bestrahlung einer Polymerfolie (3) durch mindestens ein hochbeschleunigtes Ion, um einen Ionenweg durch die Folie zu bilden;
- Ätzen des Ionenwegs von einer ersten Seite (13) der Polymerfolie (3);
- Trocknen der geätzten Folie (3);
- Abscheiden einer elektrischleitfähigen Schicht (14) auf einer zweiten Seite (11) der Polymerfolie unter Verringerung einer Öffnung (10) auf der zweiten Seite der Polymerfolie;
- Wiederöffnen der Öffnung (10) auf einen vorbestimmten Durchmesser durch Ätzen der leitfähigen Schicht (14) von der ersten Seite (13).

2. Verfahren nach Anspruch 1, wobei ein einzelnes Bismut-Ion auf eine Energie im Bereich von 10 bis 15 MeV beschleunigt und zur Polymerfolie (3) hin abgestrahlt wird, um den Ionenweg zu bilden.

3. Verfahren nach Anspruch 1 oder 2, wobei der Ionenweg durch eine alkalische Lösung geätzt wird.

4. Verfahren nach Anspruch 3, wobei die alkalische Lösung 9M NaOH umfasst.

5. Verfahren nach einem der Ansprüche 1 bis 4, wobei der Ionenweg bei Raumtemperatur geätzt wird.

6. Verfahren nach einem der Ansprüche 1 bis 5, wobei die Abscheidung ausgeführt wird, indem ein Metall oder ein Halbleiter auf die zweite Seite (11) gesputtert wird.

7. Verfahren nach einem der Ansprüche 1 bis 6, wobei die zweite Seite (11) der Folie (3) vor dem Ätzen des Ionenwegs aufgerauht wird.

8. Verfahren nach einem der Ansprüche 1 bis 7, wobei die Folie in eine elektrolytische Zelle eingefügt wird, die aus zwei Zellhälften besteht, die mit einer KF-Lösung gefüllt sind und durch die Folie geteilt sind, um den Ionenweg zu ätzen, wobei die Folie hermetisch an die beiden Zellhälften abgedichtet ist.

9. Verfahren nach einem der Ansprüche 1 bis 8, wobei ein leitfähiges Band an die leitfähige Schicht (14) angebracht wird bevor die Wiedereröffnung der Öffnung (10) ausgeführt wird.

10. Verfahren nach Anspruch 9, wobei die Folie, die auf ihrer zweiten Seite (11) durch ein leitfähiges Band bedeckt ist, erneut in die elektrolytische Zelle eingebracht wird, deren Zellhälften nun mit NaF gefüllt sind.

## Revendications

1. Procédé de fabrication d'un nanodispositif (1) comprenant les étapes :
- d'irradiation d'une feuille polymérique (3) par au moins un ion à forte accélération pour former une trace ionique à travers ladite feuille ;
- de gravure chimique de ladite trace ionique depuis une première face (13) de la feuille polymérique (3) ;
- de séchage de ladite feuille gravée chimiquement(3) ;
- de dépôt d'une couche conductrice électriquement (14) sur une seconde face (11) de la feuille polymérique diminuant ainsi une ouverture (10) au niveau de la seconde face de la feuille polymérique ;
- de réouverture de ladite ouverture (10) à un diamètre prédéterminé par la gravure chimique de ladite couche conductrice (14) depuis la première face (13).

2. Procédé selon la revendication 1, dans lequel un seul ion bismuth est accéléré à une énergie allant de 10 à 15 MeV et irradié vers ladite feuille polymérique (3) pour former ladite trace ionique.

3. Procédé selon la revendication 1 ou la revendication 2, dans lequel ladite trace ionique est gravée chimiquement par une solution caustique.

4. Procédé selon la revendication 3, dans lequel ladite solution caustique comprend 9m NaOH.

5. Procédé selon l'une quelconque des revendications 1 à 4, dans lequel ladite trace ionique est gravée chimiquement à température ambiante.

6. Procédé selon l'une quelconque des revendications 1 à 5, dans lequel ledit dépôt est effectué en pulvérisant un métal ou un semi-conducteur sur ladite seconde face (11).

7. Procédé selon l'une quelconque des revendications 1 à 6, dans lequel ladite seconde face (11) de ladite feuille (3) est grattée avant la gravure chimique de ladite trace ionique.

8. Procédé selon l'une quelconque des revendications 1 à 7, dans lequel ladite feuille est insérée dans une cellule électrolytique composée de deux moitiés de cellule remplies d'une solution KF et divisées par ladite feuille pour graver chimiquement ladite trace ionique, dans lequel ladite feuille est scellée hermétiquement sur les deux moitiés de cellule.

9. Procédé selon l'une quelconque des revendications 1 à 8, dans lequel un ruban conducteur est fixé sur la couche conductrice (14) avant l'exécution de ladite réouverture de ladite ouverture (10).

10. Procédé selon la revendication 9, dans lequel ladite feuille couverte sur sa seconde face (11) par un ruban conducteur est réintroduite vers ladite cellule électrolytique, lesquelles moitiés de cellule sont maintenant remplies de NaF.
